(19) Europäisches Patentamt
European Patent Office
Office européen des brevets

(11) EP 1 253 134 B1

(12) **EUROPEAN PATENT SPECIFICATION**

(45) Date of publication and mention of the grant of the patent:
**26.11.2008 Bulletin 2008/48**

(51) Int Cl.:
***C07C 69/54*** *(2006.01)* ***C08F 120/20*** *(2006.01)*
***C08F 220/20*** *(2006.01)* ***A61L 27/16*** *(2006.01)*
***A61L 33/06*** *(2006.01)* ***A61L 29/04*** *(2006.01)*

(21) Application number: **02008795.3**

(22) Date of filing: **19.04.2002**

(54) **Vinyl-polymerizable monomer having tertiary hydroxyl group and polymer**

Vinyl-polymerisierbare Monomere mit einer tertiären Hydroxygruppe und deren Polymere

Monomères vinyliques polymerisables ayant des groupes hydroxyle tertiaires et leur polymères

(84) Designated Contracting States:
**DE FR GB IT NL**

(30) Priority: **26.04.2001 JP 2001129047**
**17.10.2001 JP 2001319113**
**17.10.2001 JP 2001319117**

(43) Date of publication of application:
**30.10.2002 Bulletin 2002/44**

(73) Proprietor: **MITSUBISHI GAS CHEMICAL COMPANY, INC.**
**Chiyoda-ku,**
**Tokyo (JP)**

(72) Inventors:
- **Saegusa, Nobuya,**
**Mitsubishi Gas Chemical Co., Inc.**
**Hiratsuki-shi,**
**Kanagawa (JP)**
- **Honma, Akihiro,**
**Mitsubishi Gas Chemical Co., Inc.**
**Hiratsuki-shi,**
**Kanagawa (JP)**
- **Yamada, Hajime,**
**Mitsubishi Gas Chemical Co., Inc.**
**Hiratsuki-shi,**
**Kanagawa (JP)**
- **Kawashima, Takamasa,**
**Mitsubishi Gas Chemical Co.,**
**Hiratsuki-shi,**
**Kanagawa (JP)**
- **Kuwahara, Shojiro,**
**Mitsubishi Gas Chemical Co.,**
**Hiratsuki-shi,**
**Kanagawa (JP)**

(74) Representative: **Gille Hrabal Struck Neidlein Prop Roos**
**Patentanwälte**
**Brucknerstrasse 20**
**40593 Düsseldorf (DE)**

(56) References cited:
**DE-A- 10 008 367** **US-A- 3 957 362**
**US-A- 4 002 699** **US-A- 4 279 795**
**US-A- 4 524 092**

- **DATABASE CROSSFIRE BEILSTEIN [Online] Beilstein Institut zur Förderung der Chemischen Wissenschaften, Frankfurt am Main, DE; Database accession no. BRN 1851394, 1904459, 1853786 XP002208961 & IKEDA, C. K.: J. ORG. CHEM., vol. 29, 1964, pages 286-90,**
- **DATABASE CA [Online] CHEMICAL ABSTRACTS SERVICE, COLUMBUS, OHIO, US; AHMAD, H.: "Solubility parameter values of hydroxy polymers through its components an chemical group contribution technique" retrieved from STN Database accession no. 93:205171 CA XP002208962 & J. OIL COLOUR CHEM. ASSOC. (1980), 63(7), 263-70 , 1980,**
- **DATABASE CA [Online] CHEMICAL ABSTRACTS SERVICE, COLUMBUS, OHIO, US; HIGUCHI, KOHEI ET AL: "Anaerobically- and UV-curable hydroxymethylbutyl (meth)acrylates" retrieved from STN Database accession no. 111:154520 CA XP002208963 & JP 01 034947 A (KYOWA GAS CHEMICAL INDUSTRY CO., LTD., JAPAN) 6 February 1989 (1989-02-06)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DATABASE CA [Online] CHEMICAL ABSTRACTS SERVICE, COLUMBUS, OHIO, US; NOGUCHI, SHIZUO ET AL: "Anaerobically curable adhesive compositions." retrieved from STN Database accession no. 108:205981 CA XP002208964 & JP 62 265314 A (KYOWA GAS CHEMICAL INDUSTRY CO., LTD., JAPAN) 18 November 1987 (1987-11-18)
- DATABASE CA [Online] CHEMICAL ABSTRACTS SERVICE, COLUMBUS, OHIO, US; FU, S. C. ET AL: "The kinetics of a negative-tone acrylic photoresist for 193-nm lithograph" retrieved from STN Database accession no. 132:315730 CA XP002208965 & JOURNAL OF POLYMER SCIENCE, PART A: POLYMER CHEMISTRY (2000), 38(6), 954-961 , 2000,
- DATABASE CA [Online] CHEMICAL ABSTRACTS SERVICE, COLUMBUS, OHIO, US; MIZUKURA, NOBORU ET AL: "Silver halide color photographic material containing polymer coupler" retrieved from STN Database accession no. 109:219516 CA XP002208966 & JP 63 091659 A (KONICA CO., JAPAN) 22 April 1988 (1988-04-22)
- DATABASE CA [Online] CHEMICAL ABSTRACTS SERVICE, COLUMBUS, OHIO, US; OJI PAPER CO., LTD., JAPAN: "Lithographic plate by electrophotography" retrieved from STN Database accession no. 103:224445 CA XP002208967 & JP 60 107041 A (OJI PAPER CO., LTD., JAPAN) 12 June 1985 (1985-06-12)
- DATABASE CA [Online] CHEMICAL ABSTRACTS SERVICE, COLUMBUS, OHIO, US; NISHI, MASAHARU ET AL: "Syntheses and polymerizations of some.beta.-hydroxyalkyl acrylates" retrieved from STN Database accession no. 70:97517 CA XP002208968 & KOBUNSHI KAGAKU (1968), 25(284), 850-5 , 1968,
- DATABASE CROSSFIRE BEILSTEIN [Online] Beilstein Institut zur Förderung der Chemischen Wissenschaften, Frankfurt am Main, DE; BERGMANN; HERMAN: Database accession no. BRN 1757722 XP002208969 & J. APPL. CHEM., 3; 1953, 42, 48,
- DATABASE CROSSFIRE BEILSTEIN [Online] Beilstein Institut zur Förderung der Chemischen Wissenschaften, Frankfurt am Main, DE; Database accession no. BRN 1883979 XP002208970 & MAGUET, L, LERER, M.: BULL. CHEM. SOC. CHIM. FR., 1965, pages 3262-66,
- DATABASE CROSSFIRE BEILSTEIN [Online] Beilstein Institut zur Förderung der Chemischen Wissenschaften, Frankfurt am Main, DE; Database accession no. BRN 7019417 XP002208971 & ADAM, WALDEMAR ET AL.: J. AM. CHEM. SOC., vol. 116, no. 17, 1994, pages 7581-87,
- DATABASE CROSSFIRE BEILSTEIN [Online] Beilstein Institut zur Förderung der Chemischen Wissenschaften, Frankfurt am Main, DE; Database accession no. BRN 8550890 XP002208972 & EGAMI, YUICHIRO ET AL.: HETEROCYCLES, vol. 52, no. 2, 2000, pages 583-86,
- DATABASE CROSSFIRE BEILSTEIN [Online] Beilstein Institut zur Förderung der Chemischen Wissenschaften, Frankfurt am Main, DE; Database accession no. BRN 1849717 XP002208973 & CASTRO, B.: BULL. SOC. CHIM., 1967, pages 1540-47,
- DATABASE CROSSFIRE BEILSTEIN [Online] Beilstein Institut zur Förderung der Chemischen Wissenschaften, Frankfurt am Main, DE; Database accession no. BRN 1857123, 2235923 XP002208974 & SKVORTSOV, YU. M. ET AL.: J. ORG. CHEM. USSR (ENGL. TRANS.), vol. 7, no. 2, 1971, pages 224-6,
- DATABASE CA [Online] CHEMICAL ABSTRACTS SERVICE, COLUMBUS, OHIO, US; WASHIDA, HIROSHI ET AL: "Manufacture of carboxyl-containing fluorocopolymers from hydroxy-containing fluoropolymers and dibasic anhydrides" retrieved from STN Database accession no. 124:30645 CA XP002208975 & JP 07 216020 A (ASAHI GLASS CO LTD, JAPAN) 15 August 1995 (1995-08-15)
- DATABASE CA [Online] CHEMICAL ABSTRACTS SERVICE, COLUMBUS, OHIO, US; SENOO, MASAHIDE ET AL: "Positive-working radiation-sensitive resist composition" retrieved from STN Database accession no. 136:142611 CA XP002208976 & JP 2002 031891 A (TORAY INDUSTRIES, INC., JAPAN) 31 January 2002 (2002-01-31)


Remarks:

The file contains technical information submitted after the application was filed and not included in this specification

## Description

BACKGROUND OF THE INVENTION

1. Field of the Invention

The present invention relates to a medical material comprising a functional polymer having a tertiary hydroxyl group, which is produced by the polymerization of the monomer alone or the copolymerization of the monomer with various vinyl comonomers.

Because of having the tertiary hydroxyl group, the functional polymer simultaneously has a moderate reactivity and hydrophilicity, and therefore, the water content of its hydrogel can be regulated as desired by controlling the content of the tertiary hydroxyl group. The polymer is useful as a medical material because the hydrophilicity of its surface can be easily controlled.

2. Description of the Prior Art

It has been conventionally known that the hydrophilicity of a polymer can be improved by polymerizing a vinyl-polymerizable monomer having a hydroxyl group to introduce hydroxyl groups to polymer side chains. By reacting with the introduced hydroxyl groups, various types of other functional groups can be introduced. For example, a copolymer of a monomer having a primary hydroxyl group such as 2-hydroxyethyl methacrylate and a copolymer of a monomer having a secondary hydroxyl group such as 2-hydroxypropyl methacrylate have been widely used as a paint material and a medical material by utilizing their hydrophilicity and reactivity due to the primary or secondary hydroxyl group.

A polymer having a primary or secondary hydroxyl group, however, may cause problems due to the high reactivity of the hydroxyl group. For example, a polyurethane paint has been widely used as a two package paint comprising a main component including a coating resin and a hardener including polyisocyanate, which are mixed with each other just before its use. Because of an extremely high reactivity of the isocyanate groups of the hardener, the isocyanate groups rapidly react with the primary or secondary hydroxyl groups of the coating resin after mixing the main component and the hardener, thereby causing a problem of a short pot-life (usable life).

A highly hydrophilic homopolymer is water-soluble and water-swelling. Therefore, for the use in an aqueous condition, a copolymerization of a hydrophobic monomer is practically necessary. To improve the hydrophilicity, a polymer is required to have a large number of hydrophilic groups. The polymer, however, becomes soluble or swelling as the number of the primary or secondary hydroxyl groups increases to cause problems such as a dissolution of the polymer into a contacting aqueous medium, a poor appearance of a coating film surface, and a lowered mechanical strength of a coating film. Thus, no sufficient performance is obtained in the use under an aqueous condition.

As a vinyl-polymerizable compound having a tertiary hydroxyl group, pinacol derivatives are known from old. In addition, Japanese Patent Publication No. 7-061980, discloses monoesters of hydroxyadamantane. However, the proposed compounds are expensive and poor in the heat stability and the resistance to hydrolysis because of the presence of an ester linkage derived from a tertiary hydroxyl group, thereby largely limiting their application. Although a high heat resistance and a high refractive index attributable to the adamantane structure are recognized, nothing is reported up to the present on the properties attributable to the tertiary hydroxyl group.

Copolymers made by copolymerization of a hydrophilic monomer from the group of dihydroxyalkylacrylates and methacrylates and a substantially water insoluble monomer from the group of alkyl acrylates and methacrylates are described in US -A- 3,957,362. The preferred copolymer is made from 2,3-dihydroxypropyl methacrylate and methyl methacrylate. The material is used for the preparation of contact lenses.

Hydrogels are described in US -A- 4,279,795. The hydrogels are made from a hydrophilic polymer which may be prepared from a hydrophilic monomer such as hydroxyethyl acrylate of hydroxypropyl acrylate or other acrylic or methacrylic ester compounds with a free primary alcohol.

SUMMARY OF THE INVENTION

An object of the present invention is to provide a vinyl-polymerizable monomer that imparts a moderate reactivity and hydrophilicity to a polymer, thereby solving the problems mentioned above.

The inventors have found that a vinyl polymer produced by polymerizing a vinyl-polymerizable monomer having a tertiary hydroxyl group has a moderate reactivity and hydrophilicity, and have accomplished the invention based on this finding.

Thus, the present invention relates to

(1) a novel medical material wherein the surface directly contacting human body or blood is made of a polymer

having a tertiary hydroxyl group which does not contain an ester linkage derived from a tertiary hydroxyl group, which is produced by polymerizing a first monomer component of at least one vinyl-polymerizable monomer and a second monomer component of at least one vinyl comonomer other than the vinyl-polymerizable monomer, the first monomer component being 5 to 100 mol% and the second monomer component being 0 to 95 mol%, each being based on the total of the first monomer component and the second monomer component; and wherein 5 - 100 mol% of repeating units is a unit derived from the first monomer and 0-95 mol% of the repeating units is a unit derived from the second monomer component, and

the first monomer component is represented by the following formula 1:

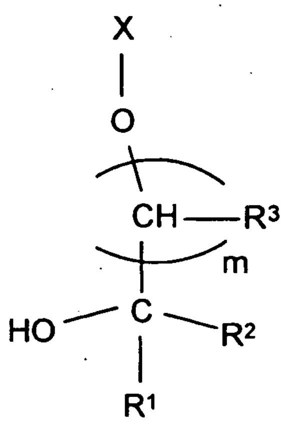

(1)

wherein X is a vinyl-polymerizable functional group; $R^1$ and $R^2$ may be the same or different and each is an alkyl group having 1 to 4 carbon atoms; $R^3$ is methyl or hydrogen; and m is an integer of 1 to 3; with the proviso that two or three $R^3$ groups when m is 2 or 3 are the same or different from each other;

(2) the medical material according to (1), wherein the number average molecular weight calibrated by polystyrene standard is 1000 to 100,000, the hydroxyl value is 5 to 300 mg KOH/g, and the ratio of the tertiary hydroxyl group to total hydroxyl groups in the polymer is 5 to 100 mol%;

(3) the medical material according to (1) or (2), wherein the polymer is in the form of a hydrogel;

(4) the medical material according to (3), wherein the water content of the hydrogel is 10 to 90% by weight;

(5) the medical material according to any of (1), (2), (3) and (4), wherein the static contact angle of 25°C water against the surface is 30 to 80°;

(6) the medical material according to any (1) to (5), wherein the first monomer is 2-hydroxy-2-methylmethacrylate;

(7) use of the medical material as defined in any of (1) to (5) as a membrane for blood lavage.

The vinyl-polymerizable group X is preferably represented by the following Formula 2:

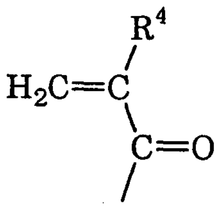

(2)

wherein $R^4$ is methyl or hydrogen. Preferred are vinyl-polymerizable monomers represented by the following Formulas 3, 4 and 5:

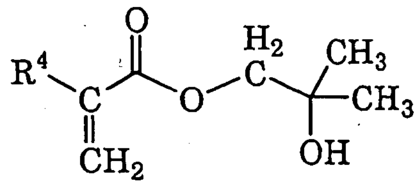

(3)

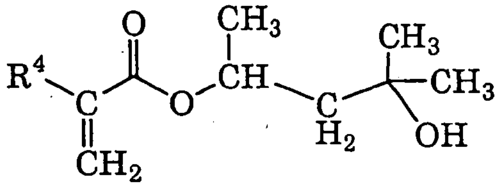

(4)

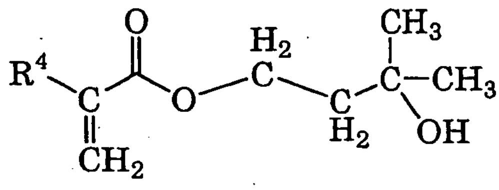

(5)

wherein $R^4$ is the same as defined above.

BRIEF DESCRIPTION OF THE DRAWINGS

Fig. 1 is a chart for showing $^1$H-NMR spectra of the vinyl-polymerizable monomer prepared in Example 1;
Fig. 2 is a chart for showing $^{13}$C-NMR spectra of the vinyl-polymerizable monomer prepared in Example 1;
Fig. 3 is a chart for showing $^1$H-NMR spectra of the vinyl-polymerizable monomer prepared in Example 3;
Fig. 4 is a chart for showing $^{13}$C-NMR spectra of the vinyl-polymerizable monomer prepared in Example 3;
Fig. 5 is a chart for showing $^1$H-NMR spectra of the vinyl-polymerizable monomer prepared in Example 5;
Fig. 6 is a chart for showing $^{13}$C-NMR spectra of the vinyl-polymerizable monomer prepared in Example 5;
Fig. 7 is a chart for showing $^1$H-NMR spectra of the vinyl-polymerizable monomer prepared in Example 6;
Fig. 8 is a chart for showing $^{13}$C-NMR spectra of the vinyl-polymerizable monomer prepared in Example 6;
Fig. 9 is a chart for showing $^1$H-NMR spectra of the vinyl-polymerizable monomer prepared in Example 7;
Fig. 10 is a chart for showing $^{13}$C-NMR spectra of the vinyl-polymerizable monomer prepared in Example 7;
Fig. 11 is a chart for showing $^1$H-NMR spectra of the vinyl-polymerizable monomer prepared in Example 8; and
Fig. 12 is a chart for showing $^{13}$C-NMR spectra of the vinyl-polymerizable monomer prepared in Example 8.

DETAILED DESCRIPTION OF THE INVENTION

The vinyl-polymerizable monomer in the medical material of the present invention is structurally characterized by:

(1) having at least one tertiary hydroxyl group;
(2) having at least one vinyl-polymerizable group; and
(3) having no ester linkage derived from a tertiary hydroxyl group. The vinyl-polymerizable monomer is synthesized by the reaction of a starting compound A for introducing the vinyl-polymerizable group and a starting compound B for introducing the tertiary hydroxyl group. The bonding residue of the starting compound A constitutes the vinyl-polymerizable group X.

The starting compound A and the starting compound B may be bonded through an ester linkage, an ether linkage, an acid anhydride linkage, an urethane linkage, etc., with the ester linkage being preferred in view of easiness of reaction.

As the starting compound A, a vinyl-polymerizable compound is usable. Various types of vinyl-polymerizable compounds such as unsaturated carboxylic acid derivatives, styrene derivatives, vinyl ethers, and allyl compounds are converted into the monomer having a tertiary hydroxyl group as far as having a reactive functional group such as hydroxyl group, ester group and carboxyl group. In view of polymerizability and easy availability, the unsaturated carboxylic acid and its ester are preferably used.

Examples of the unsaturated carboxylic acid include an aliphatic monocarboxylic acid such as acrylic acid, methacrylic acid, crotonic acid and trifluoromethylacrylic acid; an aliphatic dicarboxylic acid such as maleic acid, fumaric acid, itaconic acid and citraconic acid; and an aromatic unsaturated carboxylic acid such as cinnamic acid. These acids may be used in the form of halide. In view of easy availability and high reactivity, acrylic acid and methacrylic acid are preferred. In the present invention, acrylic acid and methacrylic acid are collectively referred to as "(meth)acrylic acid."

Examples of the ester of unsaturated carboxylic acid include an aliphatic monocarboxylate such as acrylic ester, methacrylic ester, crotonic ester and trifluoromethylacrylic ester; an aliphatic dicarboxylate such as maleic ester, fumaric ester, itaconic ester and citraconic ester; and an aromatic unsaturated carboxylate such as cinnamic ester. In view of easy availability and high reactivity, the acrylic ester and the methacrylic ester are preferably used.

In addition, an unsaturated isocyanate compound such as 2-isocyanatoethyl methacrylate and methacryloylisocyanate may be used as the starting compound A.

As the starting compound B, i.e., the other starting compound for producing the monomer, usable are a polyhydric alcohol having a primary or secondary hydroxyl group in addition to a tertiary hydroxyl group and isobutylene oxide.

Examples of the polyhydric alcohol include 2-methyl-1,2-propanediol, 2-methyl-1,2-butanediol, 2-methyl-2,3-butanediol, 3-methyl-1,3-butanediol, 2,3-dimethyl-1,2-butanediol, 2,3-dimethyl-1,3-butanediol, 2-methyl-1,2-pentanediol, 3-methyl-1,3-pentanediol, 4-methyl-1,4-pentanediol, 2-methyl-2,3-pentanediol, 2-methyl-2,4-pentanediol, 2-ethyl-1,2-butanediol, and 1,4-dihydroxy- 1-methylcyclohexane. Optical isomers, if any, may be used singly or in the form of a racemic mixture. In view of easy availability, 2-methyl-1,2-propanediol (isobutylene glycol), 2-methyl-2,4-pentanediol (hexylene glycol), and 3-methyl-1,3-butanediol are particularly preferred.

In case of using isobutylene oxide as the starting compound B, the compound of Formula 1 can be obtained by directly reacting isobutylene oxide with the starting compound A by a ring-opening addition reaction. Alternatively, an alkylene oxide such as ethylene oxide and propylene oxide is first reacted with the starting compound A by a ring-opening addition reaction, followed by the addition of isobutylene oxide at the termination stage of the ring-opening addition reaction.

The reaction to bond the starting compound A to the starting compound B is carried out in the presence of a catalyst that can be selected from various types of compounds. In case of using (meth)acrylic acid or (meth)acrylic ester as the starting compound A to carry out the reaction by esterification or ester interchange, examples of the catalysts include, but not limited to, a metal such as alkali metals, alkaline earth metals, aluminum, tin, zinc, lead, titanium, bismuth, zirconium, germanium, cobalt, chromium, iron, and copper; a compound of the preceding metal such as organometallic compounds, salts of organic acids, salts of inorganic acids, halides and hydroxides; an organic sulfonic acid; and a solid acid such as sodium methoxide, lithium methoxide, sodium aluminate, cationic ion-exchange resins, zeolites, silica-alumina, silica-titania, bentonite, montmorillonite, and activated clay. In case of using (meth)acryloyl halide as the starting compound A to carry out the reaction by esterification, usable as the catalyst are a tertiary amine and an inorganic base such as triethylamine, tripropylamine, N,N-diisopropylethylamine, tributylamine, trioctylamine, pyridine, 4-dimethylaminopyridine, 4-pyrrolidinopyridine, sodium hydroxide, potassium hydroxide, sodium carbonate, sodium hydrogencarbonate, potassium carbonate, and potassium hydrogencarbonate.

The temperature for each reaction in the presence of the above catalyst should be suitably selected. In case of using (meth)acrylic acid or (meth)acrylic ester as the starting compound A to carry out the reaction by esterification or ester interchange, the reaction is carried out at 40 to 250°C, preferably 50 to 150°C while removing alcohol and water being generated. The reaction pressure may be atmospheric, or above or below atmospheric pressure. The reaction is preferably carried out at atmospheric pressure or lower as the reaction proceeds, more preferably at 39.99 kPa (300 mmHg) or lower. To facilitate the removal of alcohol and water being generated, an azeotropic solvent may be present in the reaction system. In case of using (meth)acryloyl halide as the starting compound A to carry out the reaction by esterification, the reaction is carried out at -20 to 90°C, preferably 0 to 60°C. The reaction fails to proceed sufficiently at lower than -20°C. A temperature exceeding 90°C is unfavorable because side reactions such as polymerization are likely to occur.

The method of the present invention for producing the vinyl-polymerizable monomer having a tertiary hydroxyl group may include a step for ring-opening a cyclic ester or a cyclic dimer of oxyacid. The ring-opening reaction is carried out at 40 to 250°C, preferably 80 to 150°C optionally in the presence of the catalyst mentioned above.

The vinyl-polymerizable monomer having a tertiary hydroxyl group is easily polymerized alone or copolymerized with various vinyl comonomers by a known polymerization method such as radical polymerization, anionic polymerization and anionic coordination polymerization.

The copolymerizable vinyl comonomer may be selected from unsaturated carboxylic acids, their esters, styrene,

styrene derivatives, conjugated vinyl compounds and α-olefins. The type and the amount of the comonomer are suitably selected depending on the intended use of resultant polymer. The comonomers may be used alone or in combination of two or more. Examples of the comonomer include (meth)acrylates such as methyl (meth)acrylate, ethyl (meth)acrylate, butyl (meth)acrylate, stearyl (meth)acrylate, cyclohexyl (meth)acrylate, isobornyl (meth)acrylate, 2-methoxyethyl (meth) acrylate, 2-ethoxylethyl (meth)acrylate, 2-hydroxyethyl (meth)acrylate, and 2-hydroxypropyl (meth)acrylate; (meth)acrylates having a phospholipid-like functional group such as 2-(meth)acryloyloxyethyl phosphorylcholine; aromatic vinyl compounds such as styrene, α-methylstyrene and chlorostyrene; vinyl compounds such as acrylonitrile, methacrylonitrile, acrolein and methacrolein; α-olefins such as ethylene and propylene; N-substituted maleimides such as N-methylmaleimide, N-phenylmaleimide and N-cyclohexylmaleimide; acrylamides; vinylpyrrolidones; and (meth)acrylic acid. Also usable are polyfunctional (meth)acrylates such as ethylene glycol di(meth)acrylate, polyethylene glycol di(meth)acrylate, trimethylolpropane tri(meth)acrylate, and pentaerythritol tetra(meth)acrylate; and polyfunctional olefins such as divinylbenzene.

The polymer may be random polymer, graft polymer, block polymer, and hydrogel, although not particularly limited thereto. In whatever form the polymer may be, the characteristic features of the present invention, i.e., the moderate reactivity and hydrophilicity due to the tertiary hydroxyl group, are not lost. The proportion of the monomers is suitably selected depending on the intended use of the polymer. To ensure the effect of the tertiary hydroxyl group, the monomer of Formula 1 is preferably used in an amount of 5 to 100 mol % based on the total monomers. The polymer may be molded or formed into a shaped article or dissolved in a solvent for use as a paint for improving the surface of an article. Also, the polymer may be blended with a known resin.

The polymer in the medical material of the present invention may be produced by bulk polymerization by using only the monomers mentioned above and an polymerization initiator, or by solution polymerization, suspension polymerization or emulsion polymerization using an appropriate solvent. Examples of the solvent include alcohols such as methanol, ethanol and isopropyl alcohol; and organic solvents such as THF, DMF, dimethylsulfoxide, toluene and acetone. These solvents may be used alone or in combination of two or more in any proportion. If desired, a chain transfer agent can be used. In addition, an additive such as antioxidants, ultraviolet absorbers, lubricants, fluidity modifiers, releasing agents, antistatic agents and light diffusers; or an inorganic filler such as glass fibers, carbon fibers and clay compounds may be suitably added, if desired.

The polymer in the medical material of the present invention is first characterized by simultaneously having a moderate reactivity and a moderate hydrophilicity because of the presence of a tertiary hydroxyl group. Another distinctive characteristic is the absence of an ester linkage derived from a tertiary hydroxyl group. The polymers having a tertiary hydroxyl group which are presently known in the art are synthesized from a pinacol derivative or a hydroxyadamantane derivative to be bonded through an ester linkage derived from a tertiary hydroxyl group. With such a structure, the known polymers release a polyhydric alcohol by heating. This elimination of polyhydric alcohol becomes dominant when the temperature is elevated to 180°C or higher. Since a resin is molded or formed at over 180°C in most cases, the elimination of polyhydric alcohol sometimes causes problems such as deterioration of mechanical strength, serious discoloration and molding defect. In contrast, the above problems can be avoided in the polymer produced from the monomer because no ester linkage derived from a tertiary hydroxyl group is present therein.

Since the polymer in the medical material of the present invention is a functional resin having a moderate reactivity and hydrophilicity, the polymer is used in medical materials including contact lenses, artificial blood vessels, catheter, membranes for blood lavage and dental materials. Various molding aids such as fillers, colorants, reinforcing materials, waxes, thermoplastic polymers and oligomers can be added during the molding or forming process.

Like a known poly(2-hydroxyethyl methacrylate), the polymer of the present invention is suitable for use as a hydrogel in which the polymer retains water therein. The hydrogel is prepared by a known method. The water content largely depends on the type and the content of comonomer, and can be regulated within a desired range. A hydrogel having a water content of 10 to 90% by weight is most generally used in wide applications such as contact lenses, supports for fungus body, microorganisms and pharmacological substances, metal collectors, and cosmetic base materials.

The polymer in the medical material of the present invention has a moderate hydrophilicity, and therefore, provides a surface excellent in biocompatibility when the surface that contacts living body or blood directly is constituted by the polymer. Regarding the blood compatibility, it has been recognized that a material becomes more antithrombotic with the increase of hydrophobicity because a hydrophobic material such as silicone hardly forms thrombus. It has been afterward found that the interfacial energy between a surface and blood is reduced by grafting hydrophilic polymer chains to the surface, thereby decreasing the interaction of the surface with proteins or cells . Thus, a hydrophilic surface also prevents the adhesion of thrombus. However, in some cases, a hydrophilic surface causes minute thrombus to form embolism, damages circulating platelet, causes calcium ion deposit, or triggers the formation of thrombus.

Therefore, it is important for a medical material to be suitably balanced in hydrophilicity, hydrophobicity and biocompatibility. It is generally acknowledged that a surface having a static contact angle of 30 to 70° with 25°C water is excellent in biocompatibility. The polymer produced from the monomer of the present invention is useful because the

static contact angel with 25°C water is 30 to 80°. In addition, since the polymer in the medical material of the present invention is less soluble to water as compared with a known typical hydrophilic polymer, poly(2-hydroxyethyl (meth) acrylate), the polymer is hardly dissolved into a contact aqueous medium, hardly spoils the surface appearance and hardly reduces the mechanical strength.

The present invention will be explained in more detail by reference to the following example which should not be construed to limit the scope of the present invention.

(I) Synthesis of Vinyl-Polymerizable Monomer

EXAMPLE 1

Into a 1000-mL reactor equipped with a stirrer, a fractionating condenser, a thermometer and a gas inlet, were charged 500 g of methyl methacrylate (MMA), 222 g of 2-methyl-1,2-propanediol, 3.6 g of sodium methoxide and 0.72 g of hydroquinone. By blowing air into the mixture at 20 mL/min, the reaction was allowed to proceed at 80 to 100 °C for 6 h while distilling away the generated methanol. The removal of methanol was continued at 26.66 to 19.99 kPa (200 to 150 mmHg) for 8 h, and then the pressure was further reduced gradually to remove methanol and the unreacted methyl methacrylate by distillation.

The liquid residue was rectified under reduced pressure to obtain 375 g of a colorless transparent liquid. By GC-MAS (gas chromatography-mass spectrometry), $^{1}H$-NMR and $^{13}C$-NMR, the product was identified as 2-hydroxy-2-methylpropyl methacrylate. $^{1}H$-NMR spectra and $^{13}C$-NMR spectra are respectively shown in Figs. 1 and 2 together with assignment of peaks.

EXAMPLE 2

Into a 500-mL reactor equipped with a stirrer, a fractionating condenser, a thermometer and a gas inlet, were charged 172 g of methacrylic acid, 180 g of IBG, 1.76 g of zinc chloride and 0.35 g of 2,6-di-tert-butyl-p-cresol. By blowing air into the mixture at 20 mL/min, the reaction was allowed to proceed at 80 to 100 °C for 6 h while distilling away the generated water. The removal of water was continued at 26.66 to 19.99 kPa (200 to 150 mmHg) for 5 h, and then the pressure was further reduce gradually to further remove water by distillation.

To the liquid residue, 3.52 g of a catalyst adsorbent (Mizuka Life P-1 manufactured by Mizusawa Kagaku Kogyo Co., Ltd.). The mixture was stirred for 30 min, cooled to room temperature, and filtered to obtain 200 g of 2-hydroxy-2-methylpropyl methacrylate as a colorless transparent liquid.

EXAMPLE 3

Into a 500-mL reactor equipped with a stirrer and a thermometer, were charged 59.1 g of 2-methyl-2,4-pentanediol, 52.3 g of triethylamine and 150 mL of methylene chloride. The mixture was kept at 15 °C under stirring in a water bath. Then; 56.0 g of methacryloyl chloride was added dropwise over 15 min and then the stirring was continued for 8 h at 15 to 25 °C. After the reaction was completed, the reaction liquid was separated into aqueous layer and organic layer by adding water. The organic layer was sequentially washed with a 5% aqueous sodium hydroxide solution, a 5% hydrochloric acid, and water. After drying the organic layer over anhydrous magnesium sulfate, the solvent was removed by distillation under reduced pressure to obtain 75.7 g of a colorless transparent liquid, which was then purified by a column chromatography. By GC-MAS, $^{1}H$-NMR and $^{13}C$-NMR, the product was identified as 3-hydroxy-1,3-dimethylbutyl methacrylate. $^{1}H$-NMR spectra and $^{13}C$-NMR spectra are respectively shown in Figs. 3 and 4 together with assignment of peaks.

EXAMPLE 4

Into a 500-mL flask equipped with a thermometer, a stirrer, a reflux condenser and a dropping funnel, were charged 172 g of methacrylic acid, 0.24 g of Antage W-400 (product of Kawaguchi Kagaku Kogyo Co., Ltd.) and 2.4 g of iron(III) hydroxide. The mixture was stirred at 50 °C under heating while blowing air at 10 mL/min. From the dropping funnel, 144 g of isobutylene oxide was gradually added dropwise to the flask over 2 h. The stirring was further continued for 5 h at 60 °C under heating. The gas chromatographic analysis of the reaction liquid showed that the conversion of isobutylene oxide was 95% and the selectivity of 2-hydroxy-2-methylpropyl methacrylate was 90%. After the reaction was completed, the reaction liquid was subjected to phase separation by adding 158 g of cyclohexane and 32 g of a 2 wt % aqueous sodium carbonate to extract the target compound into the organic layer and extract the catalyst and the unreacted methacrylic acid into water layer. By removing the cyclohexane solvent under reduced pressure, 2-hydroxy-2-methylpropyl methacrylate was isolated.

EXAMPLE 5

The procedure of Example 1 was repeated except for using 430 g of methyl acrylate in place of 500 g of MMA. By GC-MAS, $^{1}$H-NMR and $^{13}$C-NMR, the product was identified as 2-hydroxy-2-methylpropyl acrylate. $^{1}$H-NMR spectra and $^{13}$C-NMR spectra are respectively shown in Figs. 5 and 6 together with assignment of peaks.

EXAMPLE 6

The procedure of Example 3 was repeated except for using 48.5 g of acryloyl chloride in place of 56 g of methacryloyl chloride. The reaction product was purified by a column chromatography. By GC-MAS, $^{1}$H-NMR and $^{13}$C-NMR, the product was identified as 3-hydroxy-1,3-dimethylbutyl acrylate. $^{1}$H-NMR spectra and $^{13}$C-NMR spectra are respectively shown in Figs. 7 and 8 together with assignment of peaks.

EXAMPLE 7

The procedure of Example 1 was repeated except for using 257 g of 3-methyl- 1,3-butanediol in place of 222 g of IBG. By GC-MAS, $^{1}$H-NMR and $^{13}$C-NMR, the product was identified as 3-hydroxy-3-methylbutyl methacrylate. $^{1}$H-NMR spectra and $^{13}$C-NMR spectra are respectively shown in Figs. 9 and 10 together with assignment of peaks.

EXAMPLE 8

The procedure of Example 1 was repeated except for using 257 g of 3-methyl-1,3-butanediol in place of 222 g of IBG, and 430 g of methyl acrylate in place of 500 g of MMA. By GC-MAS, $^{1}$H-NMR and $^{13}$C-NMR, the product was identified as 3-hydroxy-3-methylbutyl acrylate. $^{1}$H-NMR spectra and $^{13}$C-NMR spectra are respectively shown in Figs. 11 and 12 together with assignment of peaks.

(II) Polymer of Vinyl-Polymerizable Monomer Having Tertiary Hydroxyl Group EXAMPLE 9

Into a 200-mL glass reactor equipped with a stirrer, a condenser and a thermometer, were charged 60 g of 2-hydroxy-2-methylpropyl methacrylate (HBMA) as a monomer, 0.12 g of dodecanethiol (DSH) as a chain transfer, 0.3 g of 2,2'-azobis(2-methylbutyronitrile) (ABN-E) as a polymerization initiator, and 60 g of methanol as a solvent. The polymerization was allowed to proceed at 65 °C for 3 h under stirring. The polymerization liquid was dropped into diisopropyl ether to precipitate the polymer, which was then vacuum-dried. The polymerization proceeded uniformly, and the polymer thus obtained completely dissolved in an organic solvent such as methanol, acetone and THF.

The yield of the polymer was determined gravimetrically. The molecular weight was determined by a gel permeation chromatography (GPC) using THF as the developing solvent while calibrated by polystyrene standard. The contact angle as an index of hydrophilicity of the polymer was measured by a contact angle analyzer (CA-X Model manufactured by Kyowa Kaimen Kagaku Co., Ltd.). The sample was prepared by casting a polymer solution in ethanol/THF mixed solvent on a glass plate and then drying. The results are shown in Table 1.

EXAMPLE 10

Polymer was prepared in the same manner as in Example 9 except for using a monomer mixture of 39.5 g of HBMA and 25 g of MMA. The results are shown in Table 1.

EXAMPLE 11

Polymer was prepared in the same manner as in Example 9 except for using a monomer mixture of 25 g of HBMA and 37 g of MMA. The results are shown in Table 1.

EXAMPLE 12

Polymer was prepared in the same manner as in Example 9 except for using a monomer mixture of 8.8 g of HBMA and 50 g of MMA. The results are shown in Table 1.

EXAMPLE 13

Polymer was prepared in the same manner as in Example 9 except for using a monomer mixture of 4.5 g of

HBMA and 54 g of MMA. The results are shown in Table 1.

COMPARATIVE EXAMPLE 1

Polymer was prepared in the same manner as in Example 9 except for using 50 g of MMA as the monomer and toluene as the solvent. The results are shown in Table 1.

COMPARATIVE EXAMPLE 2

Polymer was prepared in the same manner as in Example 9 except for using 50 g of 2-hydroxyethyl methacrylate (HEMA) as the monomer. The results are shown in Table 1.

COMPARATIVE EXAMPLE 3

Polymer was prepared in the same manner as in Example 9 except for using a monomer mixture of 32.5 g of HEMA and 25 g of MMA. The results are shown in Table 1.

COMPARATIVE EXAMPLE 4

Polymer was prepared in the same manner as in Example 9 except for using a monomer mixture of 21 g of HEMA and 37.7 g of MMA. The results are shown in Table 1.

COMPARATIVE EXAMPLE 5

Polymer was prepared in the same manner as in Example 9 except for using a monomer mixture of 7.5 g of HEMA and 52 g of MMA. The results are shown in Table 1.

Table 1

| | Examples | | | | |
|---|---|---|---|---|---|
| | 9 | 10 | 11 | 12 | 13 |
| Ratio of charged monomers (mol%) | | | | | |
| MMA | 0 | 50 | 70 | 90 | 95 |
| HBMA | 100 | 50 | 30 | 10 | 5 |
| Yield (wt %) | 78 | 72 | 48 | 55 | 68 |
| Molecular weight (x10000) | 15 | 10 | 12 | 10 | 10 |
| Contact angle (°) | 60 | 62 | 65 | 67 | 69 |

Table 1 (contd.)

| | Comparative Examples | | | | |
|---|---|---|---|---|---|
| | 1 | 2 | 3 | 4 | 5 |
| Ratio of charged monomers (mol%) | | | | | |
| MMA | 100 | 0 | 50 | 70 | 90 |
| HEMA | 0 | 100 | 50 | 30 | 10 |
| Yield (wt %) | 67 | 77 | 56 | 53 | 62 |
| Molecular weight (x10000) | 10 | -* | 6.1 | 10 | 9.0 |
| Contact angle (°) | 72 | 60 | 61 | 65 | 68 |

*not measured because not soluble in THF.

(III) Preparation of Hydrogel

EXAMPLE 14

A uniform mixture of 8.3 g of HBMA and 0.5 g of ethylene glycol dimethacrylate (EGDMA) was added with 0.1 g of ABN-E. The resultant mixture was placed into a polypropylene tubular container with 20 mm inner diameter.

After deaerating under reduced pressure, the polymerization was allowed to proceed for 4 h in a 40 °C water tank. The polymerization was further continued for 4 h at 50 °C, for 4 h at 60 °C under heating, and then the temperature was gradually raised in a dryer from 60 °C up to 130 °C over 12 h, thereby obtaining a rod-shape polymer with about 20 mm diameter.

The rod-shape polymer was cut into a test specimen. The weight ($W_0$ g) of the test specimen in equilibrium condition on water-absorbing and the weight ($W_1$ g) of a dried test specimen were measured to calculate the water content (wt %) from the following equation:

$$\text{Water content (wt \%)} = [(W_0 - W_1)/W_0] \times 100.$$

The result is shown in Table 2.

EXAMPLES 15 to 18 and COMPARATIVE EXAMPLES 6 to 10

Each rod-shape polymer was prepared in the same manner as in Example 14 except for changing the molar ratio of the methacrylate mixture as shown in Table 2. The water content of each test specimen prepared in the same

manner is shown in Table 2.

Table 2

| | Examples | | | | |
|---|---|---|---|---|---|
| | 14 | 15 | 16 | 17 | 18 |
| Ratio of charged methacrylates (mol%) | | | | | |
| MMA | 0 | 49 | 0 | 0 | 0 |
| HEMA | 0 | 0 | 49 | 69 | 89 |
| HBMA | 99 | 50 | 50 | 30 | 10 |
| EGDMA | 1 | 1 | 1 | 1 | 1 |
| Water content (wt %) | 26 | 12 | 32 | 34 | 37 |

Table 2 (contd.)

| | Comparative Examples | | | | |
|---|---|---|---|---|---|
| | 6 | 7 | 8 | 9 | 10 |
| Ratio of charged methacrylates (mol%) | | | | | |
| MMA | 0 | 49 | 69 | 10 | 99 |
| HEMA | 99 | 50 | 30 | 89 | 0 |
| HBMA | 0 | 0 | 0 | 0 | 0 |
| EGDMA | 1 | 1 | 1 | 1 | 1 |
| Water content (wt %) | 39 | 19 | 10 | 35 | 2 |

(IV) Synthesis of Coating Resin Having Tertiary Hydroxyl Group EXAMPLE 19 (reference)

Into a 5000-mL reactor equipped with a stirrer, a condenser and a thermometer, were charged 1600 g of xylene and 400 g of butyl acetate. After raising the temperature to 85 °C, the polymerization was allowed to proceed by adding dropwise over 3 h a mixture of 500 g of styrene, 500 g of methyl methacrylate, 280 g of n-butyl acrylate, 420 g of n-butyl methacrylate, 86 g of 2-hydroxy-2-methylpropyl methacrylate, 168 g of 2-hydroxyethyl methacrylate, 14 g of acrylic acid, and 24 g of $\alpha,\alpha'$-azobisisobutyronitrile. After the dropwise addition, the stirring was continued for 2 h under heating. The stirring was further continued for 3 h under heating by adding 10 g of $\alpha,\alpha'$-azobisisobutyronitrile. By evaporating off the solvent, was obtained 1830 g of a coating resin A having a hydroxyl value of 52 mgKOH/g and an acid value of 6 mgKOH/g.

A varnish A was prepared by blending 75 parts by weight of the coating resin A and 25 parts by weight of toluene.

COMPARATIVE EXAMPLE 11

A coating resin B (1770 g) having a hydroxyl value of 52 mgKOH/g and an acid value of 5 mgKOH/g was prepared in the same manner as in Example 19 except for changing the mixture of monomers and polymerization initiator being added dropwise to a mixture of 500 g of styrene, 400 g of methyl methacrylate, 380 g of n-butyl acrylate, 420 g of n-butyl methacrylate, 240 g of 2-hydroxyethyl methacrylate, 14 g of acrylic acid, and 24 g of $\alpha,\alpha'$-azobisisobutyronitrile.

A varnish B was prepared by blending 75 parts by weight of the coating resin B and 25 parts by weight of toluene.

Each enamel paint was prepared by blending the ingredients in the proportions shown in Table 3. Specifically, a rutile titanium dioxide pigment (CR-90, product of Ishihara Sangyo Co., Ltd.) was dispersed in the varnish A or B. The dispersion was further added with a hardening agent (DN-980, product of Dainippon Ink & Chemicals, Inc.) and a leveling

agent (BYK-301, product of BYK-chemie Japan Co., Ltd.) to prepare an enamel paint. The enamel paint was coated by a doctor blade on a chemically treated steel plate in a thickness of 15 to 20 μm. The results of evaluation on the coating film are shown in Table 3.

Table 3

| | | Example 19 | Comparative Example 11 |
|---|---|---|---|
| Composition of paint (part by weight) | | | |
| Varnish | type | A | B |
| | amount | 57.2 | 57.2 |
| CR-90 | | 31.3 | 31.3 |
| DN-980 | | 11.0 | 11.0 |
| BYK-30 | | 0.5 | 0.5 |
| Pot life (h) | | 1.0 | 0.3 |
| 60° Specular gloss (%) | | 92 | 92 |
| Erichsen value (mm) | | >7 | >7 |
| Pencil hardness | | H | H |
| Rubbing test | | good | good |

The evaluations were made as follows.
Hydroxyl value: Measured according to JIS K-0070.
Acid value: Measure according to JIS K-8400.
Pot life: Time taken after the solution containing a coating resin was mixed with a hardening agent until the viscosity reached twice the initial viscosity was measured.
60° Specular gloss: Measured according to JIS K-5400.
Erichsen value: Measured according to JIS K-5400.
Pencil hardness: Measured according to JIS K-5400.
Rubbing test: After rubbing 100 times the surface of paint film with gauze impregnated with toluene, the surface was visually observed. The result was rated as "good" when no change was noticed, and "poor" when the paint film was partially dissolved.

As seen from the results, by using the tertiary hydroxyl group-containing monomer as a starting material for varnish, the pot life, as compared with using known monomers, is prolonged three times or more with the paint film performance retained.

In the present invention, the novel vinyl-polymerizable monomer of Formula 1 having a vinyl-polymerizable group X and a tertiary hydroxyl group is prepared by the reaction of a compound for introducing the vinyl-polymerizable group X and a compound for introducing the tertiary hydroxyl group. With a moderate hydrophilicity and reactivity of the monomer, a polymer produced by the copolymerization of the monomer and other vinyl comonomers is used in medical materials including contact lenses, artificial blood vessels, catheter, membranes for blood lavage and dental materials.

## Claims

1. A medical material wherein the surface directly contacting human body or blood is made of a polymer having a tertiary hydroxyl group which does not contain an ester linkage derived from a tertiary hydroxyl group,
which is produced by polymerizing a first monomer component of at least one vinyl-polymerizable monomer and a second monomer component of at least one vinyl comonomer other than the vinyl-polymerizable monomer, the first monomer component being 5 to 100 mol% and the second monomer component being 0 to 95 mol%, each being based on the total of the first monomer component and the second monomer component; and wherein
5 to 100 mol% of repeating units is a unit derived from the first monomer and 0 to 95 mol% of the repeating units is a unit derived from the second monomer component;
and the first monomer component is represented by the following formula 1:

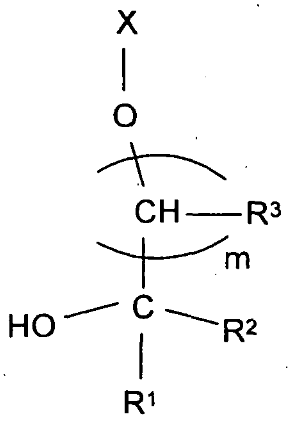

(1)

wherein X is a vinyl-polymerizable functional group; $R^1$ and $R^2$ may be the same or different and each is an alkyl group having 1 to 4 carbon atoms; $R^3$ is methyl or hydrogen; and m is an integer of 1 to 3, with the proviso that two or three $R^3$ groups when m is 2 or 3 are the same or different from each other.

**2.** The medical material according to Claim 1, wherein the number average molecular weight calibrated by polystyrene standard is 1000 to 100,000, the hydroxyl value is 5 to 300 mg KOH/g, and the ratio of the tertiary hydroxyl group to total hydroxyl groups in the polymer is 5 to 100 mol%.

**3.** The medical material according to any of Claims 1 and 2, wherein the polymer is in the form of a hydrogel.

**4.** The medical material according to Claim 3, wherein the water content of the hydrogel is 10 to 90% by weight.

**5.** A medical material according to any of the previous Claims, wherein the static contact angle of 25°C water against the surface is 30 to 80°.

**6.** The medical material according to any of the previous claims, wherein the first monomer is 2-hydroxy-2-methylpropylmethacrylate.

**7.** Use of the medical material as defined in any of Claims 1 to 5 as a membrane for blood lavage.

**Patentansprüche**

**1.** Medizinisches Material, wobei die den menschlichen Körper oder Blut direkt kontaktierende Oberfläche aus einem Polymer hergestellt ist, das eine tertiäre Hydroxylgruppe aufweist, welches keine von einer tertiären Hydroxylgruppe stammende Esterbindung enthält.
welches hergestellt wird durch Polymerisieren einer ersten Monomerkomponente aus mindestens einem vinylisch polymerisierbaren Monomer und einer zweiten Monomerkomponente aus mindestens einem von den vinylisch polymerisierbaren Monomer verschiedenen Vinylcomonomeren, wobei die erste Monomerkomponente 5 bis 100 Mol.% beträgt und die zweite Monomerkomponente 0 bis 95 Mol.% beträgt, jeweils basierend auf der Gesamtheit der ersten Monomerkomponente und der zweiten Monomerkomponente; und wobei
5 bis 100 Mol.% der wiederkehrenden Einheiten eine Einheit ist, die von dem ersten Monomer stammt, und 0 bis 95 Mol.% der wiederkehrenden Einheiten eine Einheit ist, die von der zweiten Monomerkomponente stammt;
und die erste Monomerkomponente durch die folgende Formel 1 dargestellt ist

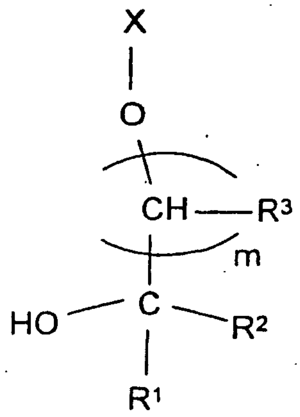

(1)

wobei X eine vinylisch polymerisierbare funktionelle Gruppe ist; $R^1$ und $R^2$ jeweils gleich oder verschieden sein können und jeweils eine Alkylgruppe mit 1 bis 4 Kohlenstoffatomen sind; $R^3$ Methyl oder Wasserstoff ist; und m eine ganze Zahl von 1 bis 3 ist, unter der Voraussetzung, dass zwei oder drei $R^3$-Gruppen, wenn m 2 oder 3 ist, gleich oder verschieden voneinander sind.

2. Medizinisches Material nach Anspruch 1, wobei das zahlenmittlere Molekulargewicht, kalibriert mit Polystyrolstandard, 1000 bis 100000 ist, der Hydroxylwert 5 bis 300 mg KOH/g ist, und das Verhältnis der tertiären Hydroxylgruppen zu den Gesamthydroxylgruppen im Polymer 5 bis 100 Mol.% beträgt.

3. Medizinisches Material nach Anspruch 1 und 2, wobei das Polymer in Form eines Hydrogels vorliegt.

4. Medizinisches Material nach Anspruch 3, wobei der Wassergehalt des Hydrogels 10 bis 90 Gew.-% beträgt.

5. Medizinisches Material nach einem der vorstehenden Ansprüche, wobei der statische Kontaktwinkel von 25°C Wasser gegen die Oberfläche 30 bis 80° beträgt.

6. Medizinisches Material nach einem der vorstehenden Ansprüche,
wobei das erste Monomer 2-Hydroxy-2-methylpropylmethyacrylat ist.

7. Verwendung des medizinischen Materials wie in einem der Ansprüche 1 bis 5 definiert, als Membran zur Blutwäsche.

## Revendications

1. Matériau médical dans lequel la surface entrant directement en contact avec le corps ou le sang humain est fait d'un polymère ayant un groupe hydroxyle tertiaire qui ne contient pas une liaison ester dérivée d'un groupe hydroxyle tertiaire,
qui est produit par la polymérisation d'un premier composant monomère d'au moins un monomère vinylique polymérisable et d'un deuxième composant monomère d'au moins un comonomère vinylique autre que le monomère vinylique polymérisable, le premier composant monomère étant présent de 5 % à 100 % en moles et le deuxième composant monomère étant présent de 0 % à 95 % en moles, chacun par rapport au total du premier composant monomère et du deuxième composant monomère ; et où
de 5 % à 100 % en moles d'unités de répétition est une unité dérivée du premier monomère et de 0 % à 95 % en moles d'unités de répétition est une unité dérivée du deuxième composant monomère ; et le premier composant monomère est représenté par la formule 1 suivante :

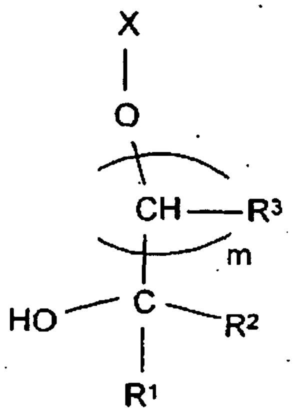

(1)

où X est un groupe fonctionnel vinylique polymérisable ; $R^1$ et $R^2$ peuvent être identiques ou différents et chacun est un groupe alkyle ayant 1 à 4 atomes de carbone ; $R^3$ est un méthyle ou un hydrogène ; et m est un entier de 1 à 3, à condition que deux ou trois groupes $R^3$ lorsque m est égal à 2 ou 3 sont identiques ou différents les uns des autres.

2. Matériau médical selon la revendication 1, dans lequel le poids moléculaire moyen en nombre calibré par la norme du polystyrène est de 1000 à 100 000, l'indice d'hydroxyle est de 5 à 300 mg KOH/g, et le rapport du groupe hydroxyle tertiaire par rapport à l'ensemble des groupes hydroxyles dans le polymère est de 5 % à 100 % en moles.

3. Matériau médical selon l'une quelconque des revendications 1 et 2, dans lequel le polymère est sous la forme d'un hydrogel.

4. Matériau médical selon la revendication 3, dans lequel la teneur en eau de l'hydrogel est de 10 % à 90 % en poids.

5. Matériau médical selon l'une quelconque des revendications précédentes, dans lequel l'angle de contact statique de l'eau à 25°C contre la surface est de 30°à 80°.

6. Matériau médical selon l'une quelconque des revendications précédentes, dans lequel le premier monomère est le 2-hydroxy-2-méthylpropylméthacrylate.

7. Utilisation du matériel médical selon l'une quelconque des revendications 1 à 5 en tant qu'une membrane de lavage du sang.

# FIG. 1

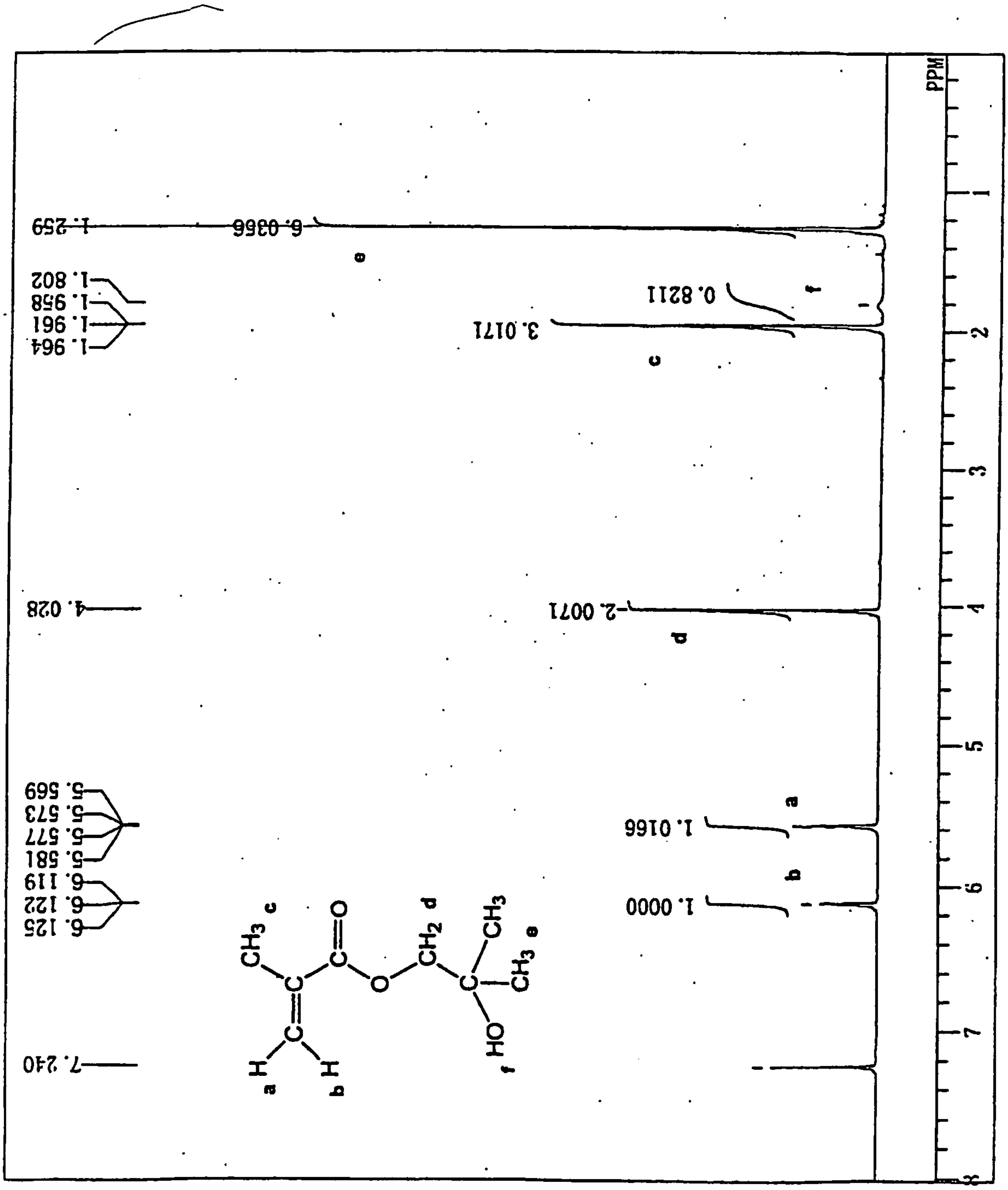
PPM
1.259
6.0366
1.802
1.958
1.961
1.964
0.8211
3.0171
4.028
2.0071
5.569
5.573
5.577
5.581
6.119
6.122
6.125
1.0166
1.0000
7.240

FIG. 2

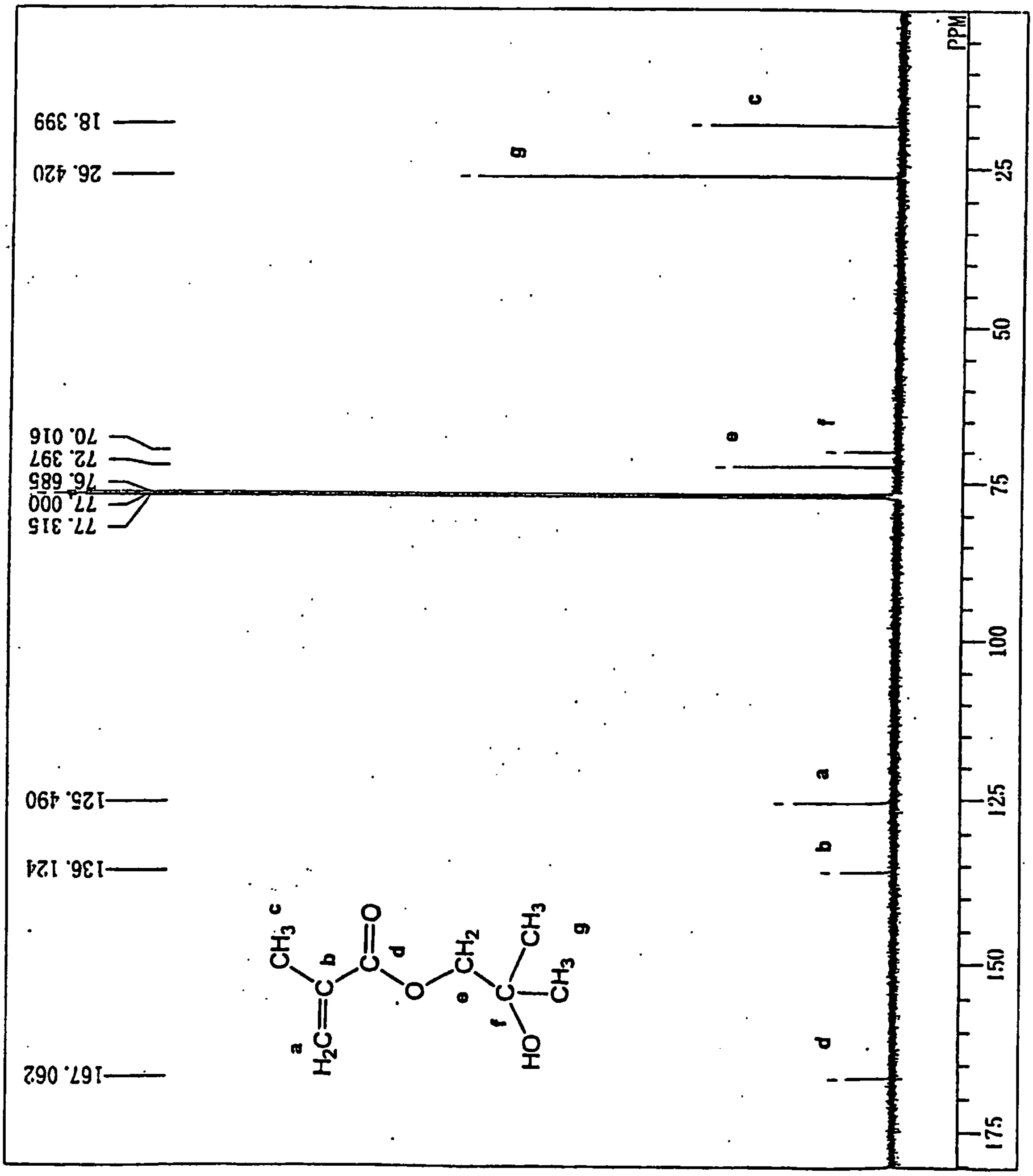
18.399
26.420
70.016
72.397
76.685
77.000
77.315
125.490
136.124
167.062
PPM
25
50
75
100
125
150
175

FIG. 3

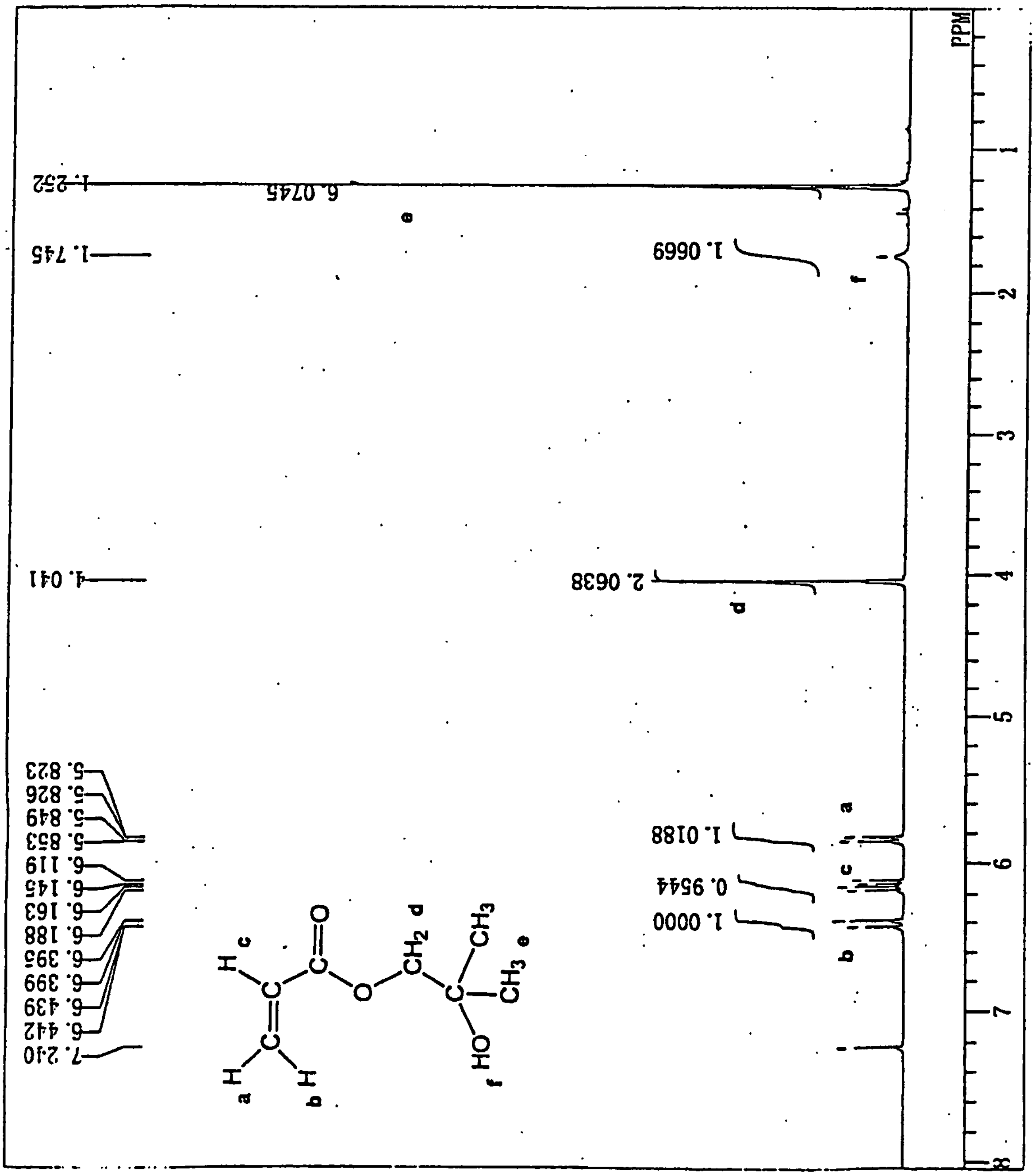
PPM
1.252
1.745
4.041
5.823
5.826
5.849
5.853
6.119
6.145
6.163
6.188
6.395
6.399
6.439
6.442
7.240
6.0745
1.0669
2.0638
1.0188
0.9544
1.0000

FIG. 4

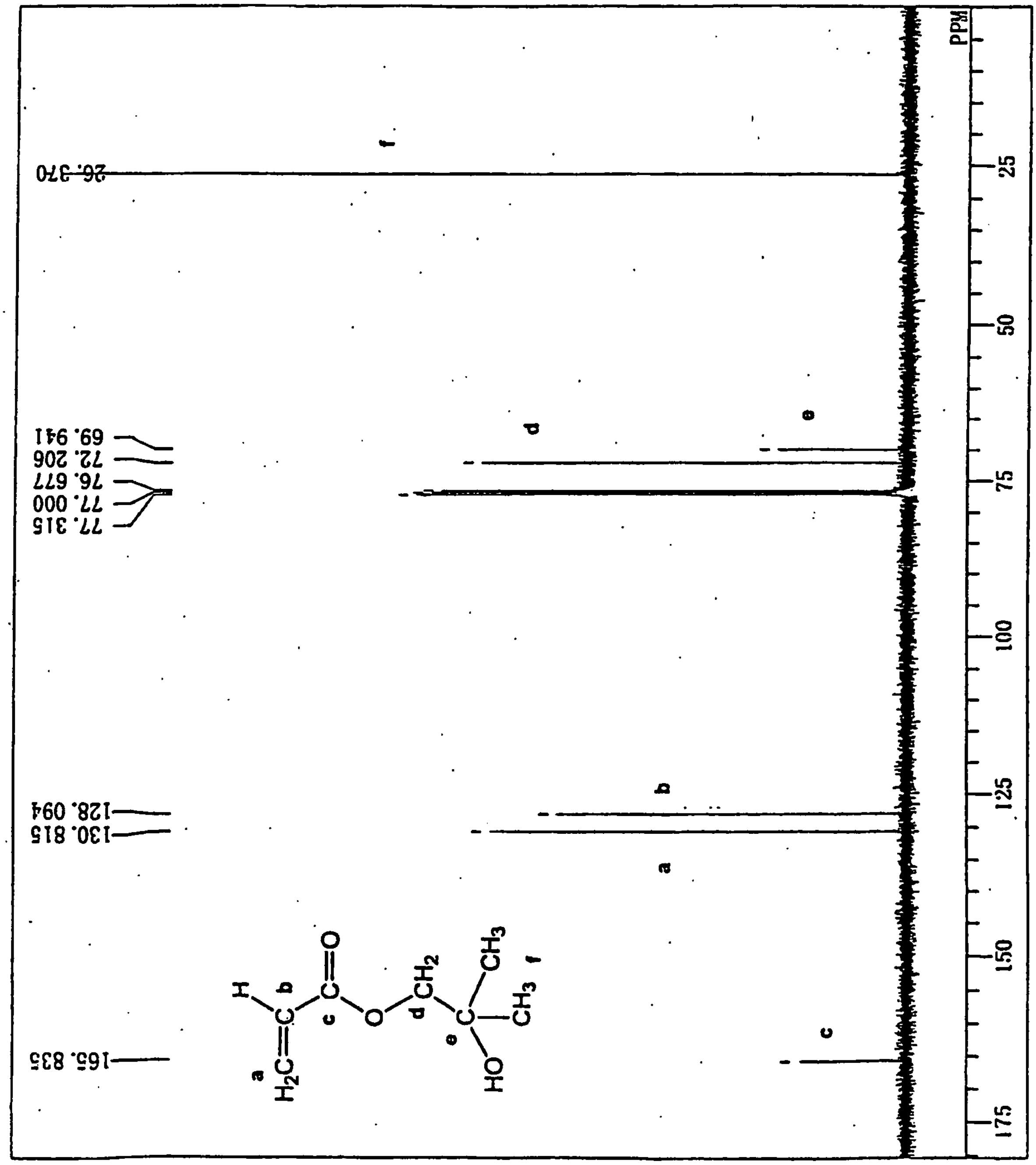
26.370
69.941
72.206
76.677
77.000
77.315
128.094
130.815
165.835
PPM
25
50
75
100
125
150
175

FIG. 5

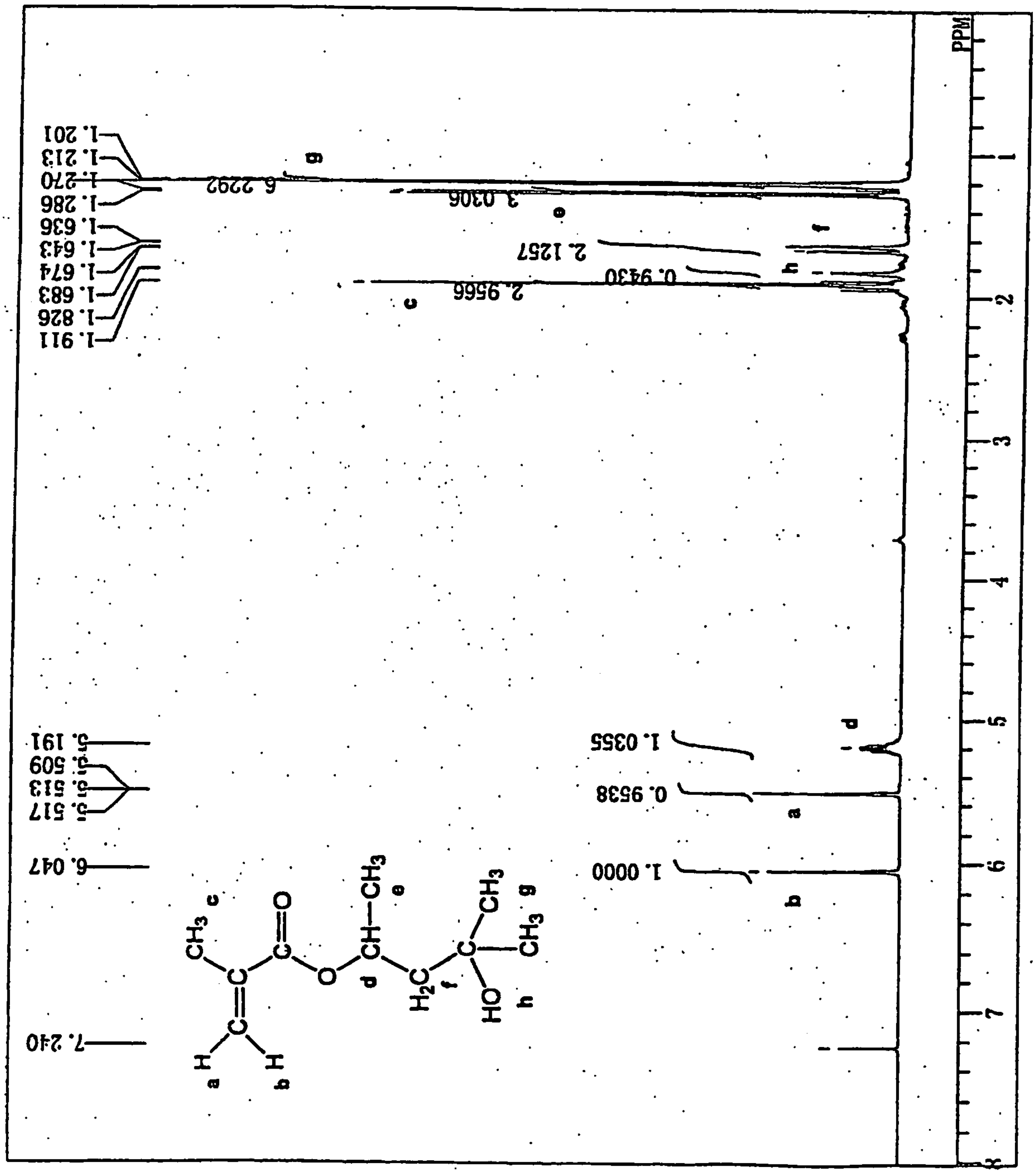
PPM
1.201
1.213
1.270
1.286
1.636
1.643
1.674
1.683
1.826
1.911
5.191
5.509
5.513
5.517
6.047
7.240
6.2292
3.0306
2.1257
0.9430
2.9566
1.0355
0.9538
1.0000

# FIG. 6

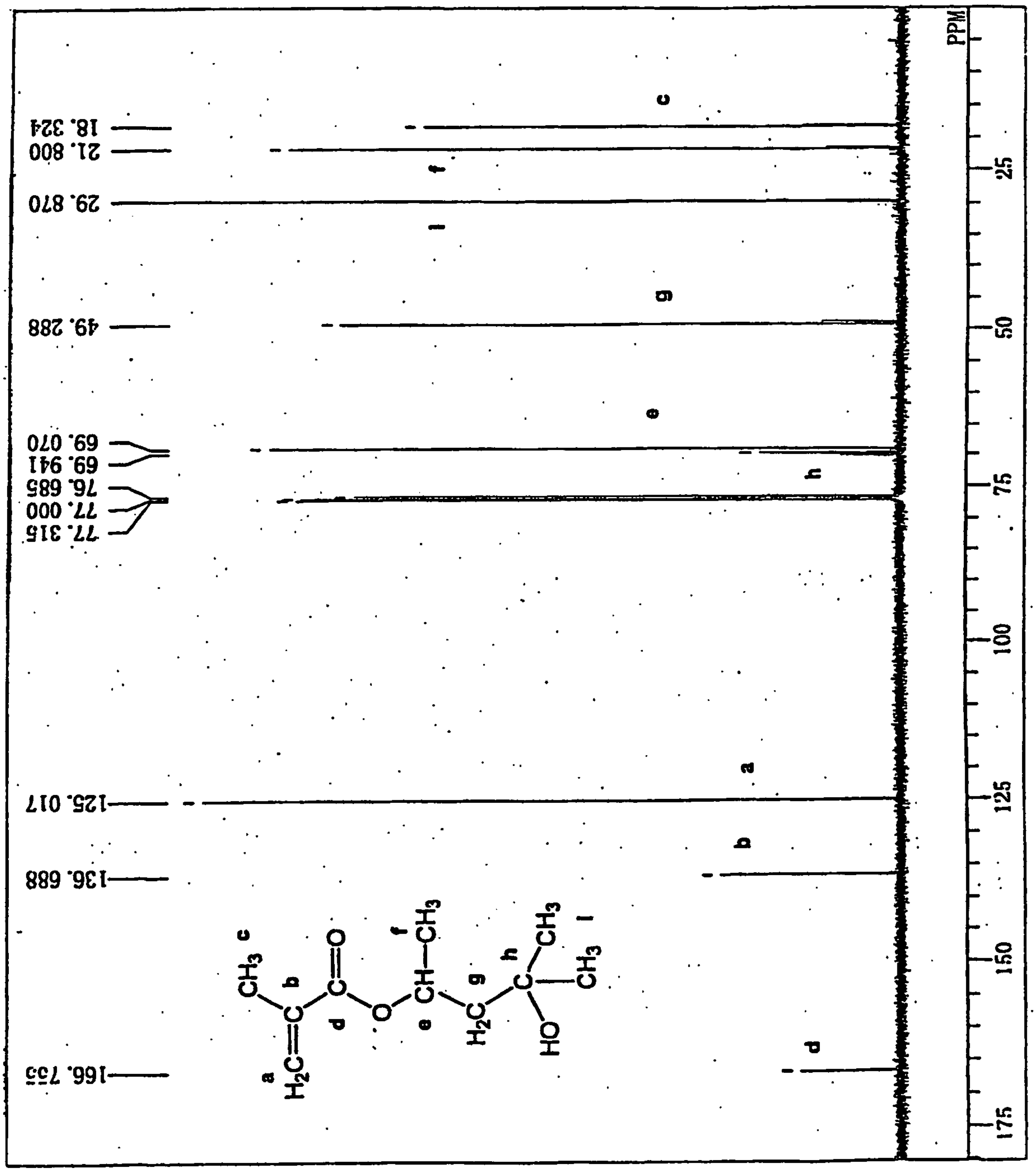

18. 324
21. 800
29. 870
49. 288
69. 070
69. 941
76. 685
77. 000
77. 316
125. 017
136. 688
166. 755
PPM
25
50
75
100
125
150
175

# FIG. 7

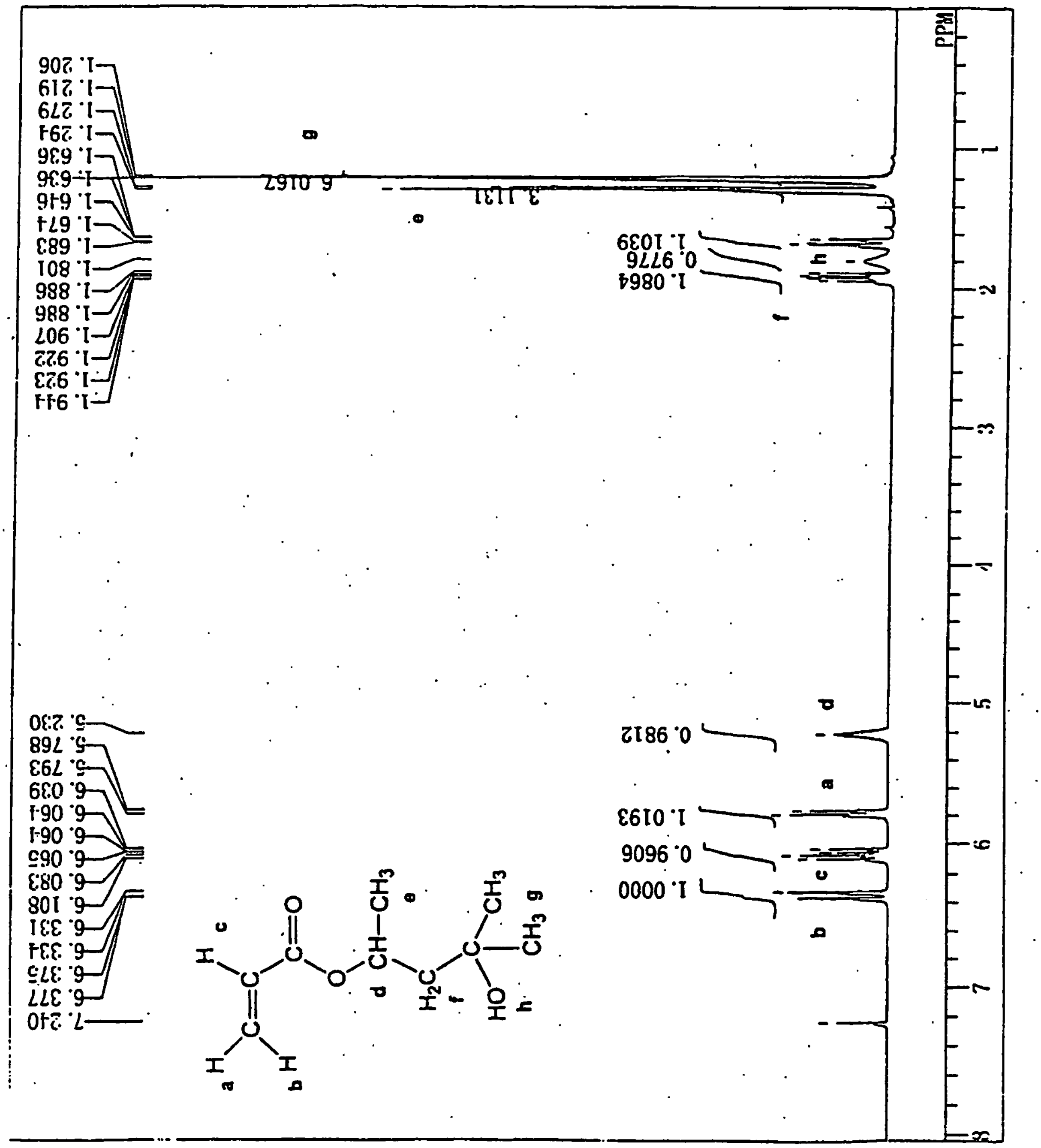
1.206
1.219
1.279
1.294
1.636
1.636
1.646
1.674
1.683
1.801
1.886
1.886
1.907
1.922
1.923
1.944
5.230
5.768
5.793
6.039
6.064
6.064
6.065
6.083
6.108
6.331
6.334
6.375
6.377
7.240
6.0162
3.1131
1.1039
0.9776
1.0864
0.9812
1.0193
0.9606
1.0000
PPM

# FIG. 8

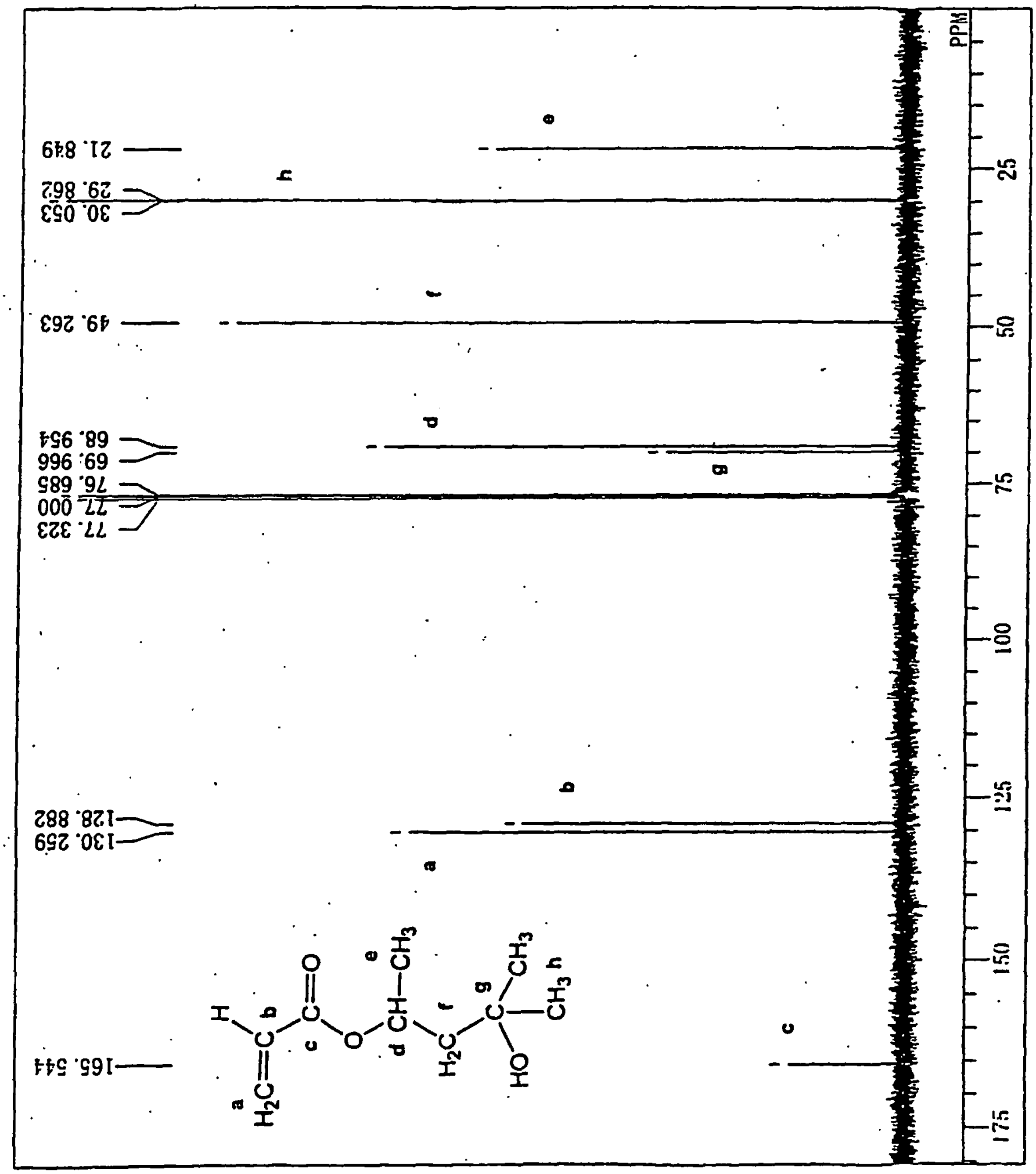
21.849
29.862
30.053
49.263
68.954
69.966
76.685
77.000
77.323
128.882
130.259
165.544
PPM
25
50
75
100
125
150
175

# FIG. 9

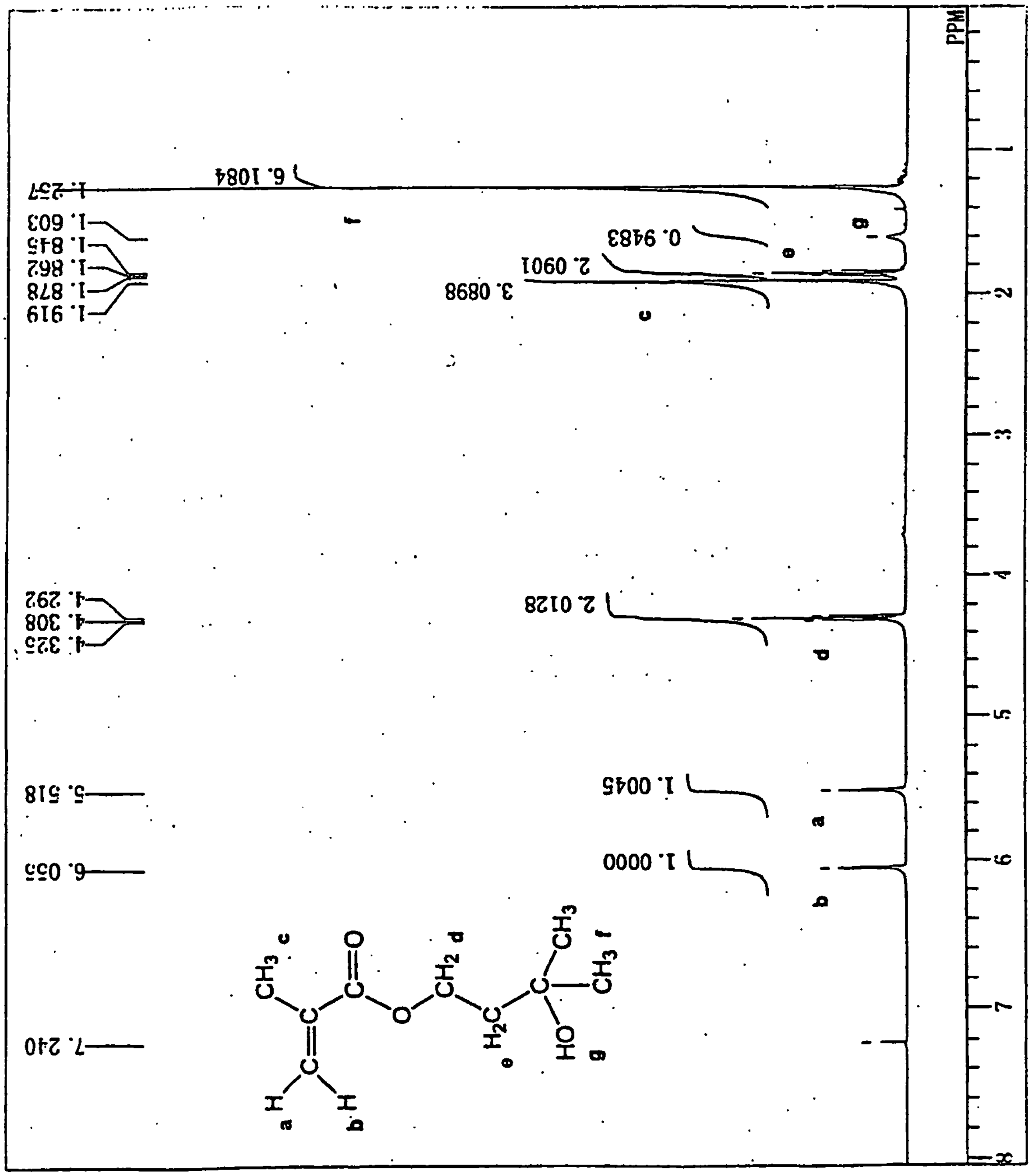
1.257
1.603
1.845
1.862
1.878
1.919
4.292
4.308
4.325
5.518
6.055
7.240
6.1084
0.9483
2.0901
3.0898
2.0128
1.0045
1.0000
PPM

# FIG. 10

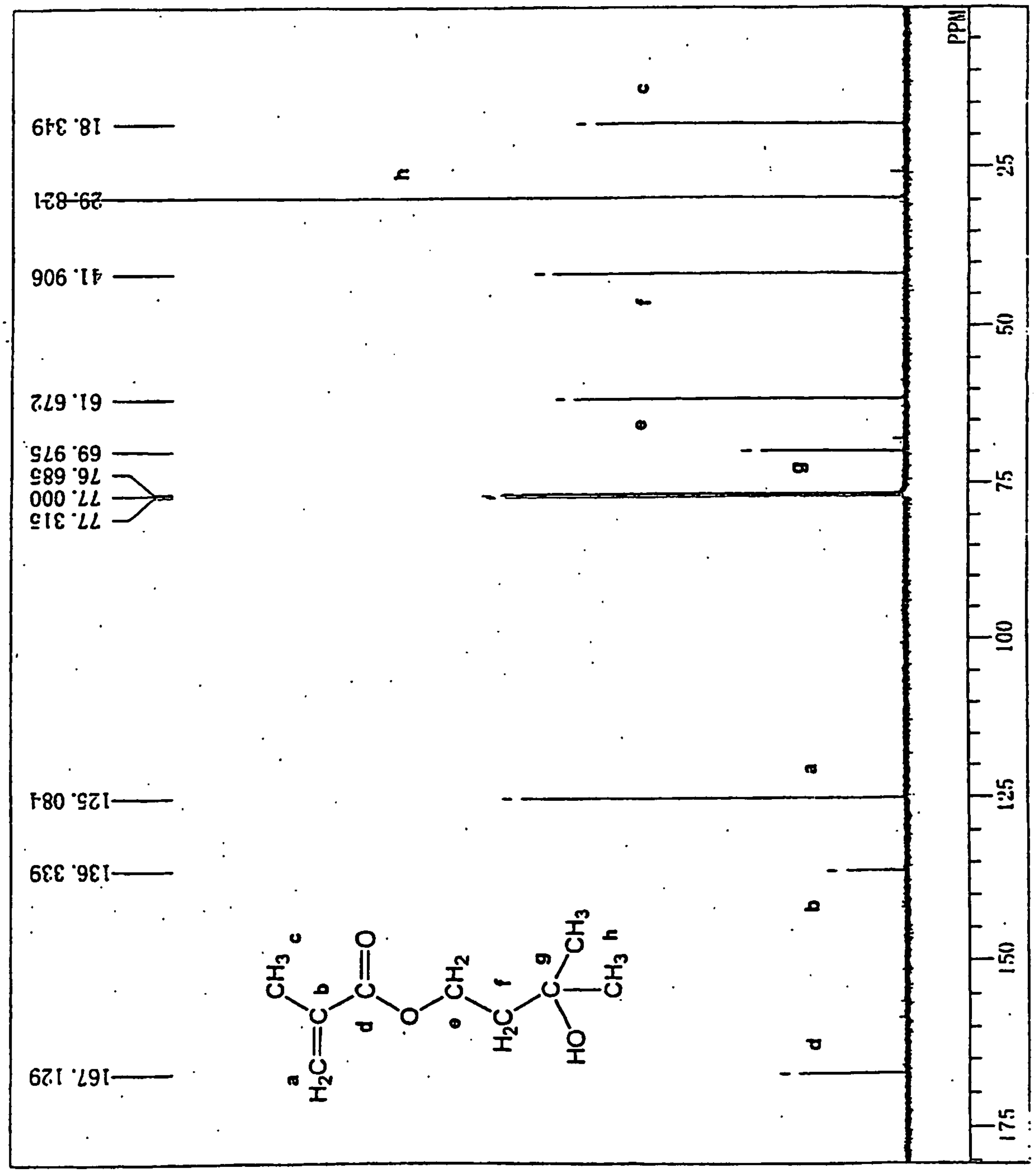
18.349
29.821
41.906
61.672
69.975
76.685
77.000
77.315
125.084
136.339
167.129
PPM
25
50
75
100
125
150
175

# FIG. 11

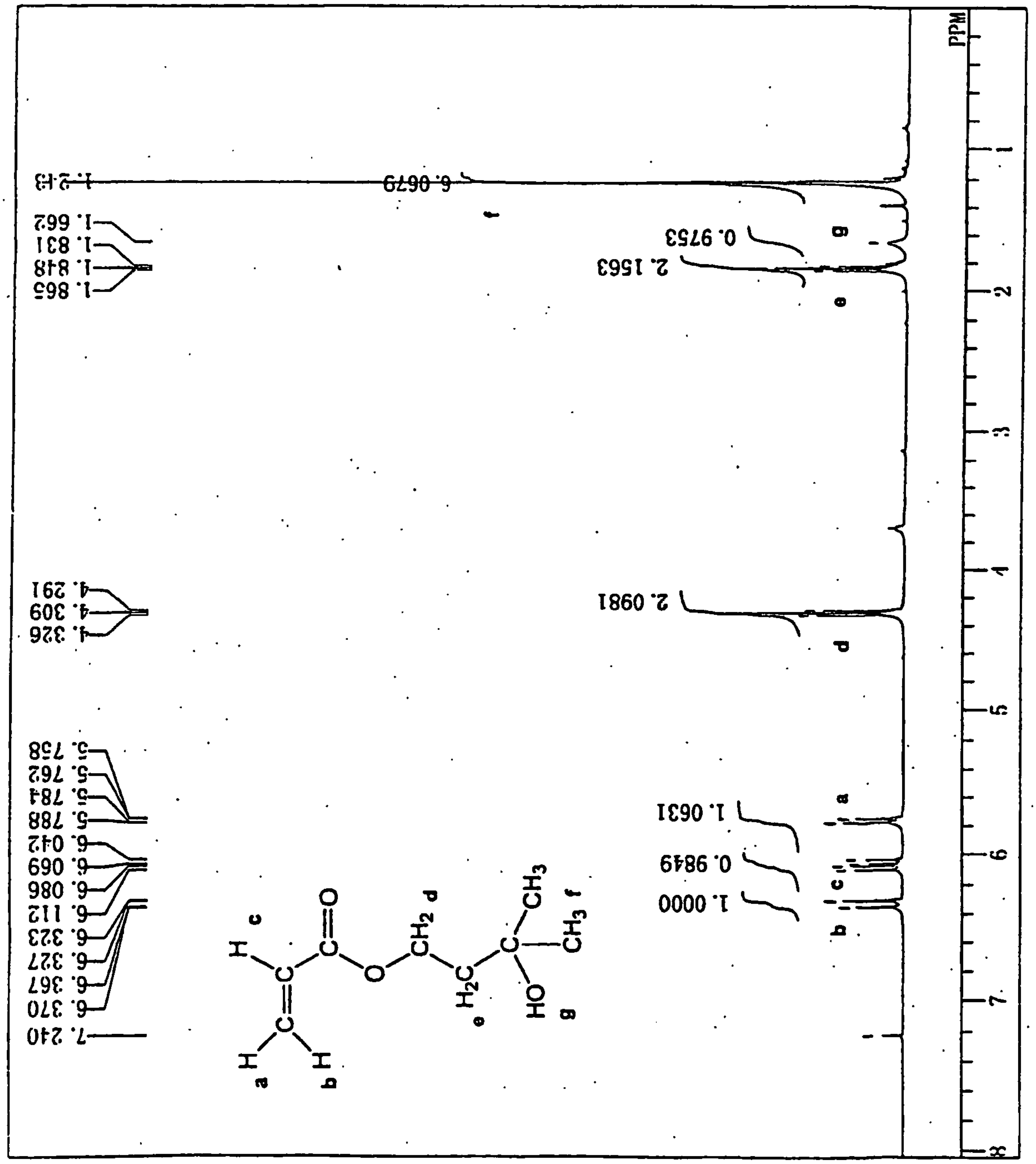
PPM
1.213
1.662
1.831
1.848
1.865
4.291
4.309
4.326
5.758
5.762
5.784
5.788
6.042
6.069
6.086
6.112
6.323
6.327
6.367
6.370
7.240
6.0679
0.9753
2.1563
2.0981
1.0631
0.9849
1.0000

# FIG. 12

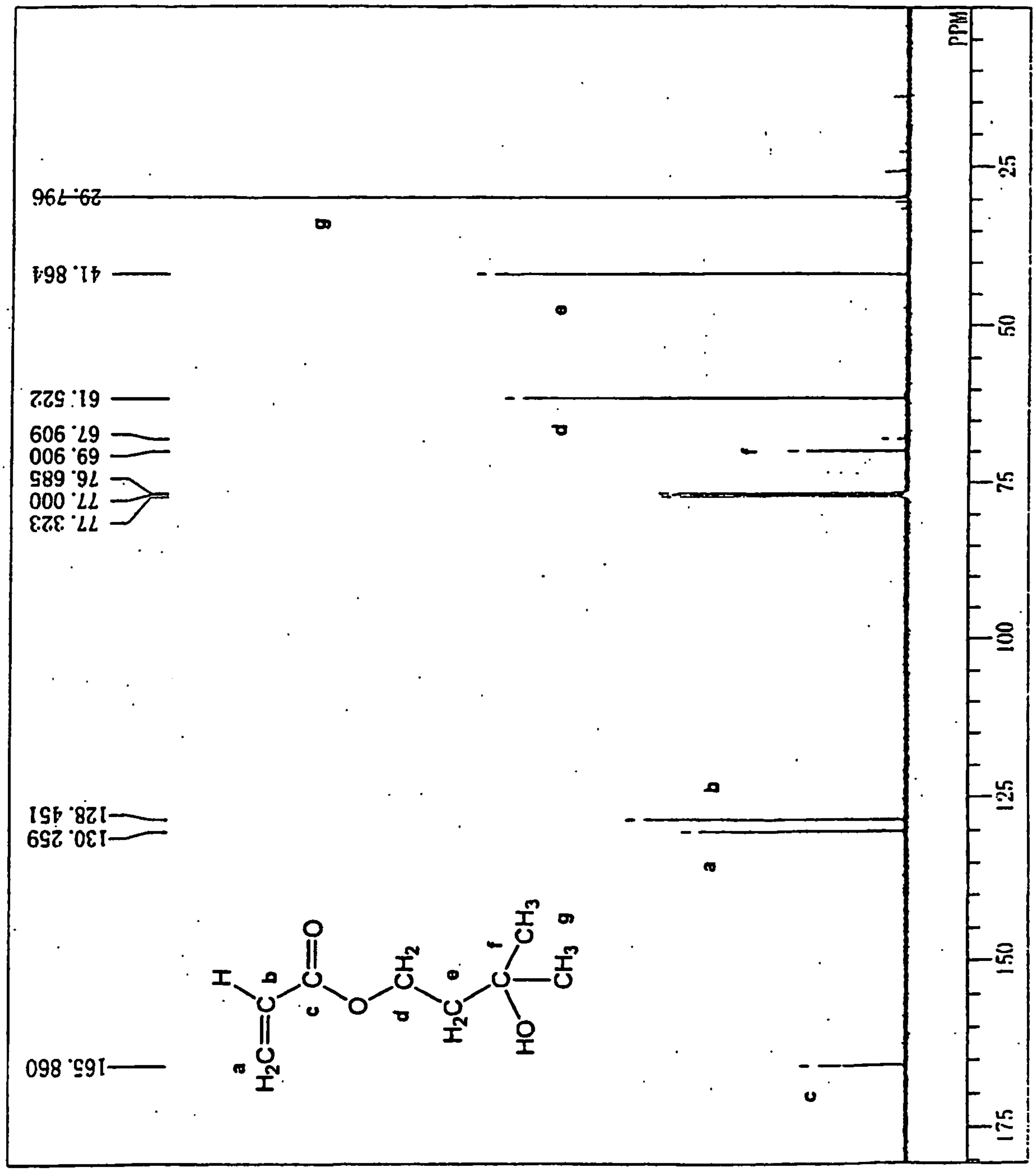

29.796
41.864
61.522
67.909
69.900
76.685
77.000
77.323
128.451
130.259
165.860
PPM
25
50
75
100
125
150
175

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 7061980 A
- US 3957362 A
- US 4279795 A